# EUROPEAN PATENT APPLICATION

(11) **EP 2 478 838 A1**
(43) Date of publication of application: **25.07.2012**
(21) Application number: 12151887.2
(22) Date of filing: 20.01.2012
(51) Int. Cl.: A61B 5/08, A61B 5/097, G01N 33/48

(54) **Liquid separator and apparatus for measuring a respiratory gas**

(30) Priority: 21.01.2011 JP 2011010658; 30.09.2011 JP 2011217214
(71) Applicant: Nihon Kohden Corporation, Shinjuku-ku Tokyo (JP)
(72) Inventor: Makino, Tetsuya, Tokyo (JP); Yamamori, Shinji, Tokyo (JP); Nonaka, Yukio, Tokyo (JP)
(74) Representative: Ilgart, Jean-Christophe

(57) **Abstract**

A liquid separator includes: a housing including: a first part including a first chamber; a second part including a second chamber; an inflow port into which a respiratory gas flows; a process chamber configured to perform a trapping process to trap liquid from the respiratory gas; and an outflow port from which the processed respiratory gas is discharged; a filter which is disposed in the process chamber interposed between the inflow port and the outflow port, and which partitions the process chamber into the first chamber on a side of the inflow port and the second chamber on a side of the outflow port; and a heating unit configured to heat at least one of the second part, the filter, and a vicinity of the filter.

## Description

### BACKGROUND OF THE INVENTION

The present invention relates to a liquid separator which is suitably used in a measurement of a respiratory gas or the like, and also to an apparatus for measuring a respiratory gas in which the liquid separator is used.

In a related-art suction technique that is called a side stream system in which a respiratory gas is guided to a measuring apparatus by using a sampling tube, there is a possibility that water vapor contained in the respiratory gas may be condensed and the condensation may enter the interior of the measuring apparatus, thereby causing the measuring apparatus to break down. In order to cope with this, a configuration is employed where a water trap which is a liquid separator is disposed in the middle of the sampling tube extending from the oral cavity to the measuring apparatus or in an input port (in front of the measuring apparatus or between the sampling tube and the measuring apparatus) of the measuring apparatus, and the condensed water is removed away.

In the related-art water trap, a filter which allows a gas to pass therethrough but prevents liquid from passing therethrough is used (refer to Patent References 1 and 2). In the case where the humidity of the supplied gas is high, when the gas reaches the downstream side (secondary side) after passing through the filter, however, there is a possibility that cooling due to adiabatic expansion may cause dew condensation in the downstream side. Therefore, the countermeasure cannot be said sufficient.

In a countermeasure against cooling condensation due to adiabatic expansion, a water fuse which absorbs a water content is disposed in the downstream side after passing through a filter. However, the water fuse is caused to expand by water absorption, and blocks a flow path, thereby disabling the measurement. Moreover, it is cumbersome to replace the water fuse, and there arises also a problem in that this may cause the cost to be increased (refer to Patent Reference 3).

In the related-art water trap, furthermore, a high-humidity respiratory gas enters as it is into a measuring apparatus, and hence there is a possibility that due condensation may occur in the measuring apparatus because of temperature variation or pressure variation in the measuring apparatus. Therefore, also the countermeasure cannot be said sufficient.

By contrast, also a related-art apparatus in which a tube that is called a Nafion tube ("Nafion" is a registered trademark) is used downstream from a water trap is provided. The tube is non-porous, exchanges water by means of diffusion which uses the hydration property of sulfonic acid, and has the property that the humidities outside and inside the tube are equalized.

When the environmental humidity is high, however, the effect of the tube is low. Moreover, the tube has substantially no effect on cool condensed water produced by adiabatic expansion in the water trap. The tube has a further problem in that aged deterioration occurs in the tube.

A related-art apparatus which employs a technique that the temperature of the interior of the apparatus is raised and the relative humidity is reduced to prevent dew condensation from occurring in the apparatus interior has a further problem in that the power consumption is large. Moreover, this technique has no effect on the cooling condensation due to adiabatic expansion in the water trap.
[Patent Reference 1] U.S. Patent No. 6,896,713 B1
[Patent Reference 2] U.S. Patent Publication No. 2009/0084383 A1
[Patent Reference 3] WO 03/035224 A1

### SUMMARY

It is therefore an object of the invention to provide a liquid separator and apparatus for measuring a respiratory gas which can cope with cooling condensation due to adiabatic expansion, and in which liquid can be prevented from entering a measuring system to cause the apparatus to break down.

In order to achieve the object, according to the invention, there is provided a liquid separator comprising: a housing including: a first part including a first chamber; a second part including a second chamber; an inflow port into which a respiratory gas flows; a process chamber configured to perform a trapping process to trap liquid from the respiratory gas; and an outflow port from which the processed respiratory gas is discharged; a filter which is disposed in the process chamber interposed between the inflow port and the outflow port, and which partitions the process chamber into the first chamber on a side of the inflow port and the second chamber on a side of the outflow port; and a heating unit configured to heat at least one of the second part, the filter, and a vicinity of the filter. The heating unit may be disposed on the second part. The heating unit may be disposed on the filter.

The heating unit may be disposed in the first chamber. The heating unit may be disposed in the first chamber to be separated from the filter.

The heating unit may be disposed in the second chamber.

The heating unit may be disposed in the second chamber to be separated from the filter.

The liquid separator may further include a cooling unit configured to cool the first part.

The cooling unit may be disposed on the first part.

The liquid separator may further include a liquid storage tank attached to the housing. A waste liquid path configured to guide the trapped liquid from the first chamber to the liquid storage tank may be formed in the housing, and the liquid separator may further include a pressure controlling unit configured to make the liquid storage tank negative with respect to the first chamber.

According to the invention, there is also provided an apparatus for measuring the respiratory gas comprising the liquid separator according to claim 1.

### BRIEF DESCRIPTION OF THE DRAWINGS

Fig. 1 is a block diagram of an apparatus for measuring a respiratory gas in which a liquid separator of an embodiment of the invention is used.
Fig. 2 is view showing the configuration of a trap in a first embodiment.
Fig. 3 is view showing the configuration of a trap in a second embodiment.
Fig. 4 is an assembly perspective view of an embodiment of the liquid separator which is configured by using a trap in a third embodiment.
Fig. 5 is an assembly perspective view of an embodiment of the liquid separator which is configured by using the trap in the third embodiment.
Fig. 6 is an assembly perspective view of an embodiment of the liquid separator which is configured by using the trap in the third embodiment.
Fig. 7 is view showing the configuration of a trap in a fourth embodiment.

### DETAILED DESCRIPTION OF EMBODIMENTS

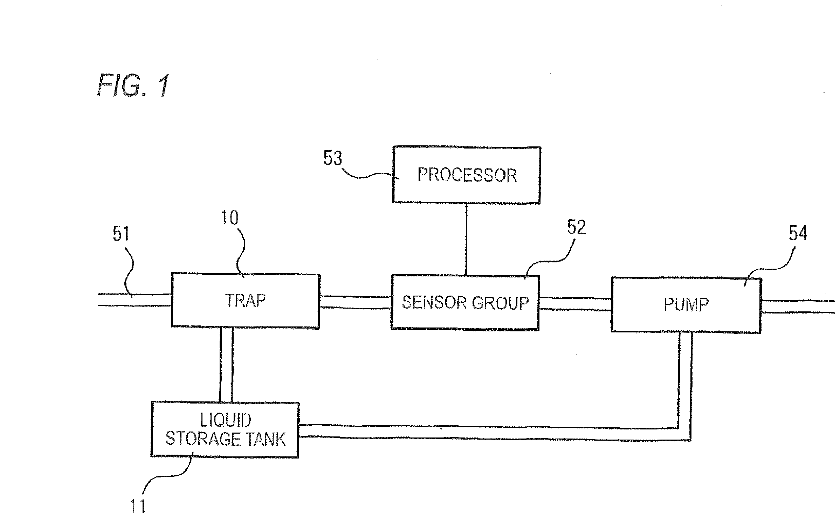

Hereinafter, embodiments of the liquid separator of the invention will be described with reference to the accompanying drawings. In the figures, the identical components are denoted by the same reference numerals, and duplicated description will be omitted. For example, the embodiments of the liquid separator are used in an apparatus for measuring a respiratory gas such as shown in Fig. 1. A sampling tube 51 of a side stream which sucks the respiratory gas of the subject is introduced into a liquid trap 10, and the respiratory gas which has undergone liquid separation is guided to a sensor group 52.

The sensor group 52 includes sensors which measure existence/non-existence, concentrations, and the like of carbon dioxide, oxygen, and anesthesia gas such as laughing gas. Signals which are detected by the sensor group 52 are sent to a processor 53, processed to be converted to display images such as graphs and numerical values, and then output from an outputting unit such as a display which is not shown.

Liquid which is condensed in the trap 10 flows into a liquid storage tank 11 to be stored therein. A configuration may be employed where the liquid storage tank 11 and the trap 10 through the sensor group 52 are sucked periodically or continuously by a pump 54. In this case, preferably, the suction is performed so that the pressure of the liquid storage tank 11 is negative with respect a first chamber of the trap 10, and the liquid which is condensed in the first chamber of the trap 10 is surely moved toward the liquid storage tank 11 and stored therein.

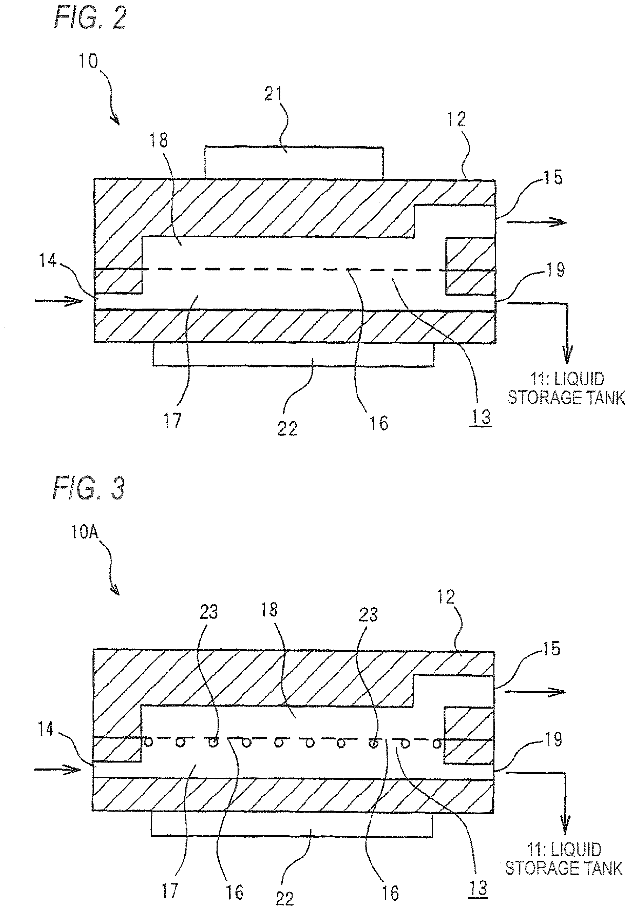

In principle, the trap 10 is configured as shown in Fig. 2, 3, or 7. Namely, the trap 10 configured by a housing 12 includes a process chamber 13 for trapping liquid from the respiratory gas. In the housing 12, an inflow port 14 into which the respiratory gas flows, and an outflow port 15 from which the processed gas is discharged are disposed, and the inflow port 14 and the outflow port 15 communicate with the process chamber 13.

A filter 16 (preferably having a gas-liquid separation function) is disposed in the process chamber while being interposed between the inflow port 14 and the outflow port 15. The process chamber 13 is partitioned by the filter 16 into a first chamber 17 which is on the side of the inflow port 14, and a second chamber 18 which is on the side of the outflow port 15. The filter 16 applies a process of trapping liquid from the respiratory gas which comes from the inflow port 14, to hold the liquid in the first chamber 17. A drain port 19 which communicates with the first chamber 17 is disposed in the housing 12, and the liquid held in the first chamber 17 flows into the liquid storage tank 11 through the drain port 19. The reference numeral 21 denotes a heater which is a heating unit. In the embodiment, the heater 21 is disposed on the outer wall of the housing 12 to heat the side of the second chamber 18, whereby the second chamber 18 is heated. Even in an environment where, when the respiratory gas passes through the filter 16, dew condensation may be caused in the second chamber 18 by cooling due to adiabatic expansion, dew condensation is prevented from occurring by the heating, and liquid can be prevented from entering a measuring system to cause the apparatus to break down.

As a cooling unit, for example, a Peltier element 22 is disposed on the outer wall of the housing 12 on the side of the first chamber 17. The Peltier element 22 cools the first chamber 17 via the outer wall of the housing 12, whereby liquid components in the respiratory gas which comes from the inflow port 14 is liquefied in the first chamber 17. The liquefied liquid flows into the liquid storage tank 11 through the drain port 19.

Fig. 3 shows a trap 10A in a second embodiment. The trap 10A of Fig. 3 in the second embodiment is different from the trap 10 of Fig. 2 in the first embodiment, in a configuration where a heater wire 23 which is a heating unit is disposed additionally in the filter 16. The other configuration is identical with that of the trap 10 of Fig. 2 in the first embodiment.

In the trap 10A having the configuration, the heater wire 23 disposed additionally in the filter 16 heats the filter 16 and the vicinity of the filter 16, and therefore the temperature of the vicinity of the place where the respiratory gas passed through the filter 16 undergoes cooling due to adiabatic expansion is high. Hence, it is possible to surely suppress dew condensation.

In the trap 10A of Fig. 3 in the second embodiment, the heater wire 23 is disposed on the filter 16 in the first chamber 17. Although not illustrated, in the trap 10A, the heater wire 23 and the filter 16 may be separately disposed.

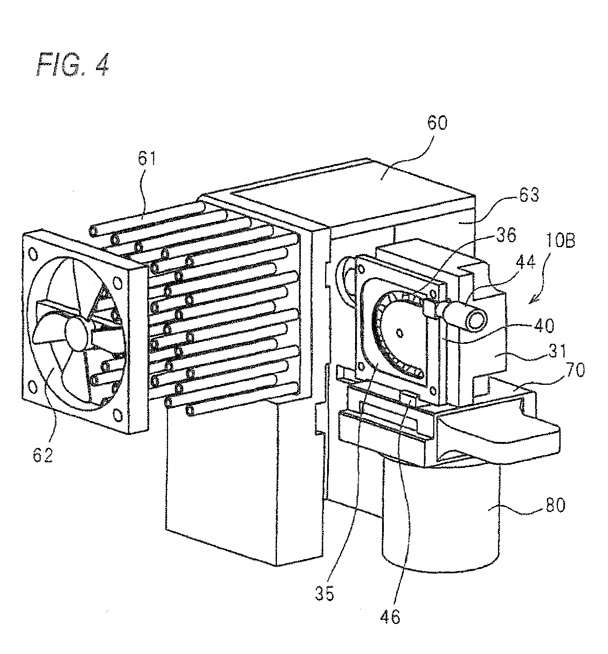

Figs. 4 to 6 show the configuration of a liquid separator including a trap 10B in a third embodiment. As shown in Fig. 4, the liquid separator of the embodiment includes a case 60 in which heat radiation fins 61 and a fan 62 are disposed on one side, and the trap 10B to which a liquid storage tank 80 is coupled through a tank holder 70.

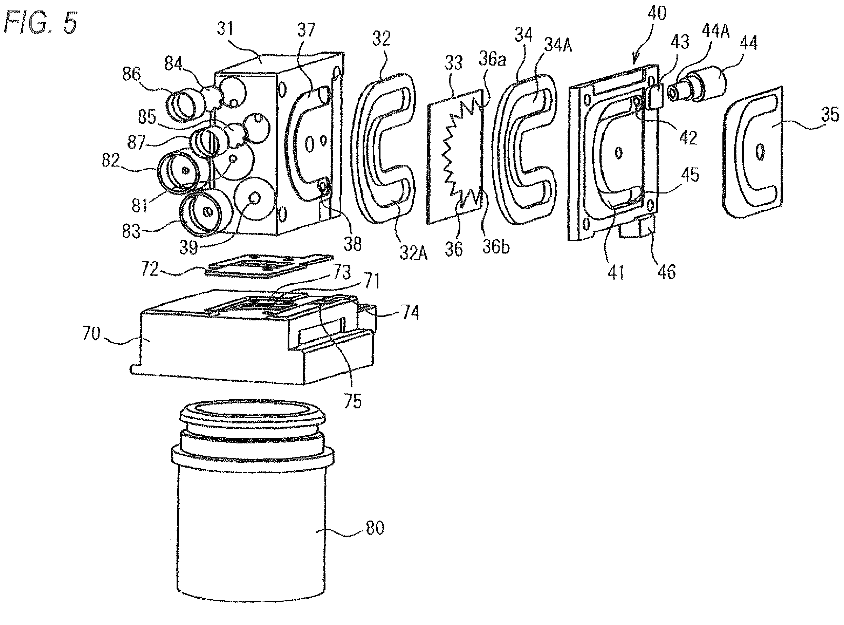

As shown in Fig. 5, the trap 10B includes a filter block 31, a packing 32, a filter 33, a packing 34, a cooling block 40, and a cover sheet 35 configured by a PET sheet. The filter 33 is preferably a gas-liquid separation filter, and configured by a member which allows a gas to pass therethrough but traps water.

The packings 32, 34 have the same shape and structure, and are configured by resin rubber plates which are formed so as to surround horseshoe-shaped openings 32A, 34A, respectively. The filter 33 is sandwiched between the packings 32, 34. A heater wire 36 is bonded in, for example, a zigzag manner to a region which is exposed through the opening 34A, in the surface of the sandwiched filter 33 that is on the side of the packing 34.

The cooling block 40 has a rectangular parallelepiped shape and a predetermined thickness. In a middle portion of the block, a horseshoe-shaped opening 41 in which a peripheral portion is covered and closed by the packing 34 is formed. An inflow port 42 for a respiratory gas is opened in an upper end portion of the opening 41. The inflow port 42 communicates with the outside through a connector receptor 43 which is formed in an upper portion of the sidewall of the cooling block 40. A lure connector 44 which is to be connected to a sampling tube of a side stream is fitted into the connector receptor 43. At this time, a tip end portion 44A of the lure connector 44 is inserted into the inflow port 42 of the connector receptor 43.

A drain port 45 through which trapped liquid is to flow is formed in a lower end portion of the opening 41. The drain port 45 communicates with the outside through a coupling portion 46 which is formed in a bottom portion of the cooling block 40.

In the filter block 31, a horseshoe-shaped groove portion 37 in which a peripheral portion is covered and closed by the packing 32 is formed in the sidewall which is opposed to the packing 32. An intake port 38 for a gas from which the liquid has been trapped is formed in a lower end portion of the groove portion 37. A gas flow path to a take-out port 39 communicates with the intake port 38 through a flow path hole formed in the filter block 31.

The tank holder 70 is a block having a substantially rectangular parallelepiped shape and a predetermined thickness. In a substantially middle portion of the block, a through hole 71 which is bored in a square columnar shape is formed. In an upper peripheral portion of the through hole 71, a groove 73 which surrounds the through hole 71, and which is used for positioning a square annular packing 72 is formed. The liquid storage tank 80 is attached to the bottom face side of the tank holder 70. The liquid storage tank 80 is detachably attached to the tank holder 70 by screws or fitting portions.

A discharge hole 74 communicating with the liquid storage tank 80 is formed in a position of the tank holder 70 which is outside the through hole 71. A groove 75 into which the coupling portion 46 formed in a bottom portion of the cooling block 40 is to be fitted is formed in the periphery of the discharge hole 74.

A hole which is opposed to the through hole 71 of the tank holder 70 is formed in a bottom portion of the filter block 31, and a flow path extending from the hole communicates with a suction outlet port 81 through an inner hole for suction. Bottomed cylindrical connector guides 82, 83 are attached to the suction outlet port 81 and the above-described take-out port 39, respectively. A tube extending to the pump 54 which has been described with reference to Fig. 1 is connected to the connector guide 82, and a tube extending to the sensor group 52 which has been described with reference to Fig. 1 is connected to the connector guide 83. A suction process is performed through the tubes. Here, the interior of the liquid storage tank 80 is configured so that the pressure is negative with respect to the opening 41 which is a process chamber that is closed by the cover sheet 35. Therefore, liquid condensed in the opening 41 surely flows toward the liquid storage tank 80.

In a state where the packings 32, 34 are clamped between the filter block 31 and the cooling block 40, the packings sandwiching the filter 33 are fixed by screwing the filter block 31 and the cooling block 40 together.

At this time, terminals 36a, 36b which are disposed both end portions of the heater wire 36 bonded to the filter 33 are contacted with electrodes which are disposed in corresponding positions of the filter block 31, and which are not shown. Lead wires extend from the electrodes which are not shown, to the surface to which the connector guides 82, 83 of the filter block 31 are attached, and are connected to electrodes 84, 85, respectively. Connector guides 86, 87 are fitted into groove portions of the disk-like electrodes 84, 85, and attached to peripheral portions of the electrodes 84, 85. Connectors of a power supply cable are connected to the connector guides 86, 87, respectively so that electric power is supplied to the heater wire 36.

In the trap 10B in an assembled state, the cover sheet 35 is bonded to a groove portion which is in an exposed face of the cooling block 40, and which corresponds to the cover sheet 35. As a result, a section of the configuration extending from the cover sheet 35 to the filter block 31 is similar to that of the trap 10A which has been described with reference to Fig. 3. The unit configured by the assembled trap 10B, the tank holder 70, and the liquid storage tank 80 is inserted from an opening 63 of the case 60 toward the rear side, to be fitted to the case 60.

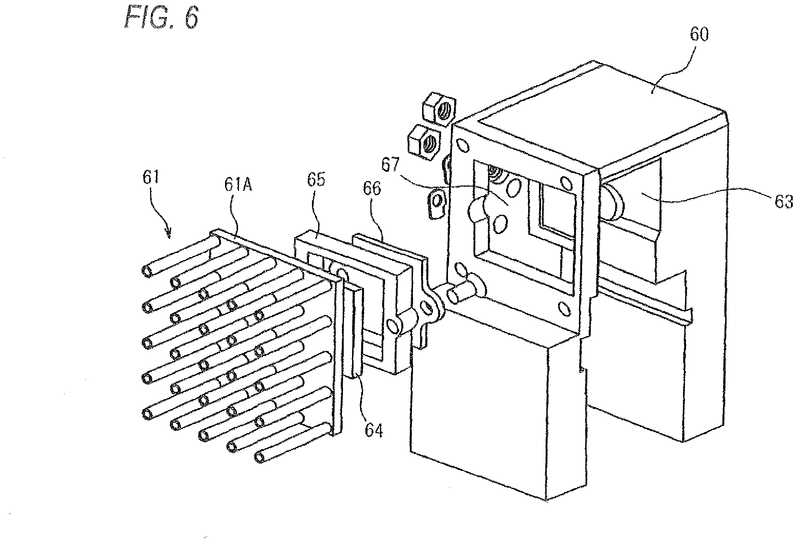

Fig. 6 shows the configuration of a portion of the case 60 to which the radiation fins 61 are attached. A planar Peltier element 64, a frame-like heat insulating member 65 having a square opening into which the Peltier element 64 is accommodated, and a metal plate 66 for heat transfer are sequentially arranged in the direction from the radiation fins 61 to the case 60. The radiation fins 61 are disposed on the hot side of the Peltier element 64, and the metal plate 66 is disposed on the side of the cold side of the Peltier element 64. The Peltier element 64, the heat insulating member 65, and the metal plate 66 are integrally fixed together with a base plate 61A of the radiation fins 61 by screws or the like, and disposed so as to, through a window 67 of the case 60, butt against the cover sheet 35 of the trap 10B.

The configuration of the track portion 10B in the embodiment can trap liquid in a similar manner as that of the track portion 10A shown in Fig. 3.

Namely, a respiratory gas which comes from the lure connector 44 enters the opening 41 of the cooling block 40, and then is subjected to cooling by the Peltier element 64, and dew condensation occurs. The liquefied liquid flows into the liquid storage tank 80 through the drain port 45. The respiratory gas in the opening 41 of the cooling block 40 reaches the groove portion 37 in the filter block 31 through the filter 33. When the respiratory gas passes through the filter 33, cooling due to adiabatic expansion is caused. In the side of the opening 41 which is a first chamber on the side of the inflow port, however, the heater wire 36 is disposed so as to be bonded to the filter 33, and therefore the filter 33 and the vicinity of the filter 33 are heated. Consequently, dew condensation does not occur despite of cooling due to adiabatic expansion which occurs when the respiratory gas passes through the filter 33. As a result, liquefied liquid does not flow to the take-out port 39 through the flow path hole formed in the filter block 31, liquid does not invade from the connector guide 83 into a measuring apparatus including the sensor group, and the dehumidified respiratory gas enters the measuring apparatus. Therefore, dew condensation can be prevented from occurring inside the measuring apparatus, and the apparatus can be protected from breaking down.

In the embodiment, the mode where the heater wire 36 is bonded to the filter 33 has been described. It is required to heat the filter 33 or the vicinity (the respiratory gas which passes through the filter 33) of the filter 33. Therefore, the heater wire 36 and the filter 33 may be separately disposed.

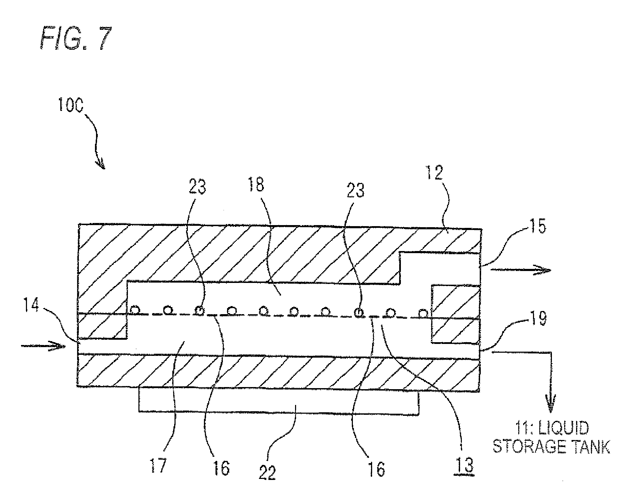

Fig. 7 shows a trap 10C in a fourth embodiment. The trap 10C of Fig. 7 in the fourth embodiment is different from the trap 10 of Fig. 2 in the first embodiment, in a configuration where a heater wire 23 which is a heating unit is disposed additionally in the filter 16, and further different from the trap 10A of Fig. 3 in the second embodiment, in a configuration where the heater wire 23 which is a heating unit is disposed additionally in the second chamber 18 of the filter 16. The other configuration is identical with that of the trap 10 of Fig. 2 in the first embodiment, and that of the trap 10A of Fig. 3 in the second embodiment. Although not illustrated, in the trap 10C, the heater wire 23 and the filter 16 may be separately disposed.

According to an aspect of the invention, the liquid separator includes the heating unit, which heats at least one of the second chamber that is formed by partitioning with the filter and that is on the side of the outflow port, the filter, and the vicinity of the filter. Even in an environment where dew condensation may be caused in the second chamber by cooling due to adiabatic expansion, dew condensation is prevented from occurring by heating, and liquid can be prevented from entering the measuring system to cause the apparatus to break down.

According to an aspect of the invention, the liquid separator has the configuration where the heating unit is disposed additionally in the filter. Therefore, heating is performed in the vicinity of the place where cooling due to adiabatic expansion is caused, and hence it is possible to surely suppress dew condensation.

According to an aspect of the invention, the liquid separator includes the cooling unit which cools the side of the first chamber. In the first chamber, therefore, a water content can be removed from a high-humidity respiratory gas, and the dehumidified respiratory gas flows into a measuring apparatus. Consequently, a situation where the apparatus is caused to break down by dew condensation inside the measuring apparatus can be surely prevented from occurring, by a small amount of power consumption.

According to an aspect of the invention, the liquid separator has the configuration where the pressure of the liquid storage tank is made negative with respect the first chamber. Therefore, liquid of condensation in the first chamber can be surely guided into the liquid storage tank, and a situation where the passage of the respiratory gas is impeded by accumulation of the liquid condensed in the first chamber can be prevented from occurring.

According to an aspect of the invention, when an apparatus for measuring a respiratory gas includes the liquid separator, it is possible to prevent the apparatus from being caused to break down by condensing of water vapor contained in the respiratory gas, and entering of the condensed water vapor into the apparatus.

## Claims

1. A liquid separator comprising:
a housing including:
a first part including a first chamber;
a second part including a second chamber;
an inflow port into which a respiratory gas flows;
a process chamber configured to perform a trapping process to trap liquid from the respiratory gas; and
an outflow port from which the processed respiratory gas is discharged;
a filter which is disposed in the process chamber interposed between the inflow port and the outflow port, and which partitions the process chamber into the first chamber on a side of the inflow port and the second chamber on a side of the outflow port; and
a heating unit configured to heat at least one of the second part, the filter, and a vicinity of the filter.

2. The liquid separator according to claim 1, wherein the heating unit is disposed on the second part.

3. The liquid separator according to claim 1, wherein the heating unit is disposed on the filter.

4. The liquid separator according to claim 3, wherein the heating unit is disposed in the first chamber.

5. The liquid separator according to claim 1, wherein the heating unit is disposed in the first chamber to be separated from the filter.

6. The liquid separator according to claim 3, wherein the heating unit is disposed in the second chamber.

7. The liquid separator according to claim 1, wherein the heating unit is disposed in the second chamber to be separated from the filter.

8. The liquid separator according to claim 1, further comprising a cooling unit configured to cool the first part.

9. The liquid separator according to claim 8, wherein the cooling unit is disposed on the first part.

10. The liquid separator according to claim 1, further comprising a liquid storage tank attached to the housing, wherein
a waste liquid path configured to guide the trapped liquid from the first chamber to the liquid storage tank is formed in the housing, and
the liquid separator further includes a pressure controlling unit configured to make the liquid storage tank negative with respect to the first chamber.

11. An apparatus for measuring the respiratory gas comprising the liquid separator according to claim 1.
